# FASCICULE DE BREVET EUROPEEN

(11) **EP 3 565 491 B1**
(45) Date de publication et mention de la délivrance du brevet: **31.05.2023**
(21) Numéro de dépôt: 18700320.7
(22) Date de dépôt: 09.01.2018
(51) Int. Cl.: A61B 17/70, A61B 17/86

(54) **VIS OSSEUSE POUR DISPOSITIF D'OSTÉOSYNTHÈSE, ENSEMBLE FORMÉ PAR UNE VIS, UN CONNECTEUR ET UN ÉCROU, ET KIT COMPORTANT AU MOINS UN TEL ENSEMBLE**
KNOCHENSCHRAUBE FÜR EINE OSTEOSYNTHESEVORRICHTUNG, ANORDNUNG AUS EINER SCHRAUBE, EINEM VERBINDER UND EINER MUTTER SOWIE KIT MIT MINDESTENS EINER SOLCHEN ANORDNUNG
BONE SCREW FOR OSTEOSYNTHESIS DEVICE, ASSEMBLY FORMED BY A SCREW, A CONNECTOR AND A NUT, AND KIT COMPRISING AT LEAST ONE SUCH ASSEMBLY

(30) Priorité: 09.01.2017 FR 1750163
(43) Date de publication de la demande: 13.11.2019
(73) Titulaire: S.M.A.I.O, 69800 Saint-Priest (FR)
(72) Inventeur: ROUSSOULY, Pierre, 69450 Saint Cyr au Mont D'Or (FR); BERNARD, Marc, 33850 Leognan (FR); DO, Antoine, 69007 Lyon (FR); CHAROSKY, Sébastien, 31320 Vieille-Toulouse (FR); DEWALD, Christopher J., Lincolnshire, Illinois 60069 (US); GEHRCHEN, Poul Martin, 2820 Gentofte (DK); SILVESTRE, Clément, 69340 Francheville (FR)
(74) Mandataire: Lavoix
(86) Numéro de dépôt international: PCT/EP2018/050425
(87) Numéro de publication internationale: WO 2018/127596

(56) Documents cités:
- WO-A1-2016/166163
- FR-A1- 2 765 093
- US-A1- 2008 195 150
- US-A1- 2012 209 332
- US-A1- 2016 183 983
- US-B1- 6 709 434

## Description

La présente invention concerne les dispositifs prothétiques implantables sur les os d'un patient, et plus particulièrement les vis osseuses destinées à fixer lesdits dispositifs.

Elle trouve une application particulière mais non exclusive aux vis osseuses destinées à être implantées sur des vertèbres et sur la tête desquelles sont disposés des éléments permettant de relier la vertèbre à un dispositif en forme de tige.

Il est classique que, dans la chirurgie du rachis, on utilise des appareillages d'ostéosynthèse dont l'élément essentiel est constitué par une ou des tiges s'étendant sur une partie plus ou moins longue du rachis pour corriger le positionnement relatif de deux ou plusieurs vertèbres. Ces tiges sont fixées sur les vertèbres par l'intermédiaire de vis osseuses, portant, à leur extrémité émergeant de la vertèbre, donc à leur partie qu'on appellera « supérieure », un élément de réception de la tige. Cet élément est, souvent, en forme de « tulipe », c'est-à-dire présentant deux parois opposées définissant une ouverture dans laquelle la tige est insérée. Ces parois portent un filetage intérieur, et après l'insertion de la tige dans l'espace défini par les parois, un bouchon fileté est vissé entre les parois pour obturer l'espace et y coincer solidement la tige.

Dans d'autres variantes qui sont celles dont relève la présente invention, ainsi que le document US2016183983, la tige est reçue non dans une telle tulipe, mais dans un connecteur qui permet de décaler latéralement la tige par rapport à l'axe longitudinal de la vis et de choisir une orientation de la tige par rapport à la vis qui est bien adaptée au traitement de la pathologie du patient. C'est alors ce connecteur qui est fixé à la vis, par insertion sur un élément en forme de tige filetée, qu'on appellera un « pion » dans la suite du texte, et qui constitue la partie supérieure de la vis. Le connecteur est fixé sur ce pion au moyen d'un écrou venant en prise avec la tige filetée du pion et pressant le connecteur contre la base du pion.

Typiquement, le pion et la partie inférieure de la vis qui porte un filetage assurant sa pénétration puis sa fixation dans la vertèbre sont placés dans le prolongement l'un de l'autre selon un même axe longitudinal et forment un ensemble monobloc.

L'insertion de la vis dans la vertèbre par le chirurgien est assurée au moyen d'un instrument tel qu'un tournevis à extrémité creuse, venant en prise avec une surface présentant des pans (formant, par exemple, un hexagone) qui est ménagée vers l'extrémité inférieure du pion, en tout cas au-dessus de l'extrémité supérieure de la partie filetée de la vis. La rotation de l'instrument assure la pénétration de la partie filetée de la vis dans la vertèbre. Une fois que la vis est fixée, le pion et son extrémité inférieure émergent de la vertèbre. On peut alors insérer sur le pion le connecteur destiné à recevoir la tige de l'appareillage d'ostéosynthèse, placer la tige dans le connecteur, et insérer l'écrou de serrage sur la partie filetée du pion de manière à bloquer l'ensemble vis-connecteur-tige dans la position souhaitée.

Avantageusement, le pion est sécable, c'est-à-dire qu'il présente à l'origine, à un certain niveau de sa longueur, une zone de moindre résistance pouvant être rompue aisément par le chirurgien. De cette façon, le pion peut, de construction, présenter une longueur initiale relativement importante qui facilite l'insertion du connecteur puis l'insertion de l'écrou. Une fois l'écrou serré, la zone de moindre résistance se retrouve un peu au-dessus de la surface supérieure de l'écrou. Le chirurgien exerce alors sur le pion un effort latéral qui conduit à briser le pion au niveau de sa zone de moindre résistance, réduisant ainsi la longueur du pion à ce qui est strictement nécessaire au fonctionnement de l'ensemble vis-connecteur-tige-écrou. De cette façon, le pion ne présente plus une longueur excessive qui serait gênante pour le patient.

Cependant, les dispositifs connus présentent une caractéristique qui, à l'usage, peut s'avérer désavantageuse. En effet, l'ensemble formé par le connecteur et l'écrou se retrouve, après l'insertion du connecteur et/ou le blocage de l'écrou, autour de la partie du pion présentant des pans, grâce à laquelle la vis a pu être fixée dans la vertèbre. De cette façon, une manipulation visant à ajuster la position longitudinale du pion par rapport à la surface de la vertèbre, par un serrage supplémentaire ou un desserrage partiel de la vis, n'est pas possible sans un démontage complet de l'ensemble connecteur-tige-écrou. Le but de l'invention est de procurer aux chirurgiens une nouvelle configuration d'une vis comportant un pion, exempte des inconvénients qui ont été signalés, c'est-à-dire qui reste pleinement accessible et réglable en hauteur même après la mise en place du connecteur, de la tige et de l'écrou.

A cet effet, l'invention a pour objet une vis osseuse comportant une partie inférieure d'ancrage osseux munie d'un filet, une partie supérieure constituée par un pion à surface externe filetée pour la réception d'un écrou, et un siège pour la réception d'un connecteur destiné à relier la vis à une tige d'un dispositif d'ostéosynthèse et à être maintenu contre le siège par l'écrou, caractérisée en ce que ledit pion est creux et présente un espace intérieur débouchant sur son extrémité supérieure, la partie inférieure dudit espace intérieur se terminant par une empreinte munie de pans pour la réception d'un tournevis.

Le pion présente une ligne de moindre résistance le rendant sécable.

L'invention a également pour objet un ensemble formé par une vis osseuse, un connecteur destiné à relier la vis à une tige d'un dispositif d'ostéosynthèse, et un écrou destiné à maintenir le connecteur contre le siège de la vis, caractérisé en ce que la vis osseuse est du type précédent.

L'écrou peut comporter dans sa partie inférieure une collerette destinée à venir reposer sur la face supérieure dudit connecteur pour le coincer contre le siège après vissage de l'écrou.

Le connecteur peut comporter une surface supérieure en forme de portion de sphère et la surface inférieure de l'écrou est alors aussi en forme de portion de sphère.

L'écrou peut comporter une partie supérieure tubulaire.

Ladite partie tubulaire de l'écrou peut comporter une ligne de moindre résistance la rendant sécable.

L'espace intérieur de l'écrou peut comporter des pans avec lesquels un tournevis peut être mis en prise lors du serrage et du desserrage de l'écrou.

Lesdits pans peuvent alors subsister sur l'espace intérieur de l'écrou après la section de sa partie supérieure tubulaire selon la ligne de moindre résistance.

L'invention a également pour objet un kit pour dispositif d'ostéosynthèse, du type comportant au moins un ensemble vis - connecteur - écrou, caractérisé en ce que au moins une vis est du type précédent, et en ce qu'il comporte également un tournevis destiné à venir en prise avec ladite empreinte terminant la partie inférieure de l'espace intérieur du pion.

Ledit écrou peut être du type selon lequel son espace intérieur comporte des pans, et ledit kit peut comporter également un tournevis destiné à venir en prise avec les pans de l'espace intérieur de l'écrou.

Comme on l'aura compris, l'invention repose sur le remplacement de la surface externe à pans habituelle du pion, permettant la préhension de la vis par le tournevis et son insertion dans l'os, par une empreinte pour la mise en prise d'un tournevis, présentant des pans ou une configuration en étoile (du type communément appelé « Torx^{®} »), cette empreinte étant située à l'intérieur du pion lui-même, et demeurant accessible en permanence même après le montage et la fixation complets de l'ensemble vis-connecteur-tige-écrou.

De plus, le fond de cette empreinte est très préférentiellement situé vers la base du pion, et l'empreinte demeure ainsi fonctionnelle même après la séparation de la partie supérieure du pion selon la ligne de moindre résistance, s'il y en a une. Il devient ainsi possible, à tout moment, de réajuster l'enfoncement de la vis dans l'os du patient, simplement par une réinsertion du tournevis à l'intérieur du pion et une rotation dudit tournevis, dans un sens ou dans l'autre.

Une autre solution qui serait conforme à l'invention serait, cependant, de conserver une surface externe à pans classique sur le pion, qui serait utilisable lors de l'implantation de la vis. L'empreinte à pans située à l'intérieur du pion s'y ajouterait, et serait utilisée lors d'ajustements postérieurs de l'enfoncement de la vis. La surface externe à pans ayant un diamètre plus grand que l'empreinte, elle permettrait l'application d'un moment de rotation relativement élevé et l'utilisation d'un tournevis de plus grand diamètre, donc une plus grande facilité d'insertion initiale de la vis dans l'os que si seule l'empreinte interne au pion était utilisée, surtout à un stade où la vis n'est pas encore ancrée solidement dans l'os. Une fois que la vis est ancrée à une position quasi-définitive, elle ne peut plus s'incliner, et il devient plus aisé de la faire tourner avec un tournevis de petit diamètre du type de celui qui sera inséré dans l'empreinte interne au pion.

L'invention sera mieux comprise à la lecture de la description qui suit, donnée en référence aux figures annexées suivantes :
- La figure 1 qui montre en vue de face une vis osseuse de l'ensemble selon l'invention ;
- La figure 2 qui montre en vue de profil le connecteur destiné à venir s'emmancher sur la vis osseuse pour lui raccorder une tige d'un dispositif d'ostéosynthèse rachidienne ;
- La figure 3 qui montre ce même connecteur en vue de dessus ;
- La figure 4 qui montre, vu de face, l'écrou qui assure le blocage du connecteur et de la tige sur la vis ;
- La figure 5 qui montre ce même écrou en perspective vu de dessous ;
- La figure 6 qui montre ce même écrou en perspective vu de dessus ;
- La figure 7 qui montre vu en perspective, l'ensemble selon l'invention formé par la vis, le connecteur et l'écrou à l'état assemblé, avec la tige du dispositif d'ostéosynthèse auquel l'ensemble selon l'invention est intégré ;
- La figure 8 qui montre vu de profil ce même ensemble avec la tige, ainsi que l'os dans lequel la vis est insérée ;
- La figure 9 qui montre vu de face en coupe longitudinale ce même dispositif, mais avec un écrou de forme classique, différent de celui représenté sur les figures 4-8.

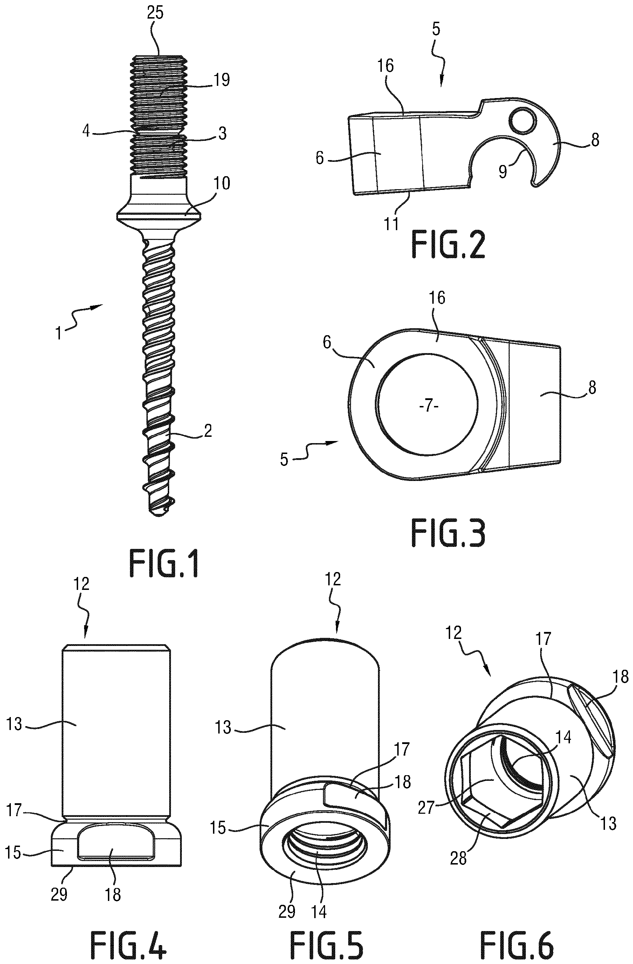

La vis osseuse 1 de l'ensemble selon l'invention comporte dans sa partie inférieure, comme il est classique, un filet 2 destiné à assurer l'ancrage de la vis 1 dans l'os auquel elle est destinée, par exemple une vertèbre comme cela sera donné en exemple non limitatif. Dans sa partie supérieure, la vis 1 comporte une tige filetée cylindrique 3 qu'on appellera un « pion », présentant de préférence, comme représenté, une ligne de moindre résistance sous forme d'une diminution locale 4 de son épaisseur. C'est sur ce pion 3 que vient s'emmancher un connecteur 5, visible sur les figures 2 et 3. Ce connecteur 5 comporte un corps 6 pourvu d'une perforation 7 qui le traverse de part en part et permet l'emmanchement du connecteur 5 sur le pion 3. Sur le corps 6 est venu de matière un appendice en forme de crochet 8 dont l'ouverture 9 est tournée vers la partie inférieure du connecteur 5.

La vis 1 comporte un siège 10 sur lequel vient se reposer, après l'emmanchement, la face inférieure 11 du connecteur 5.

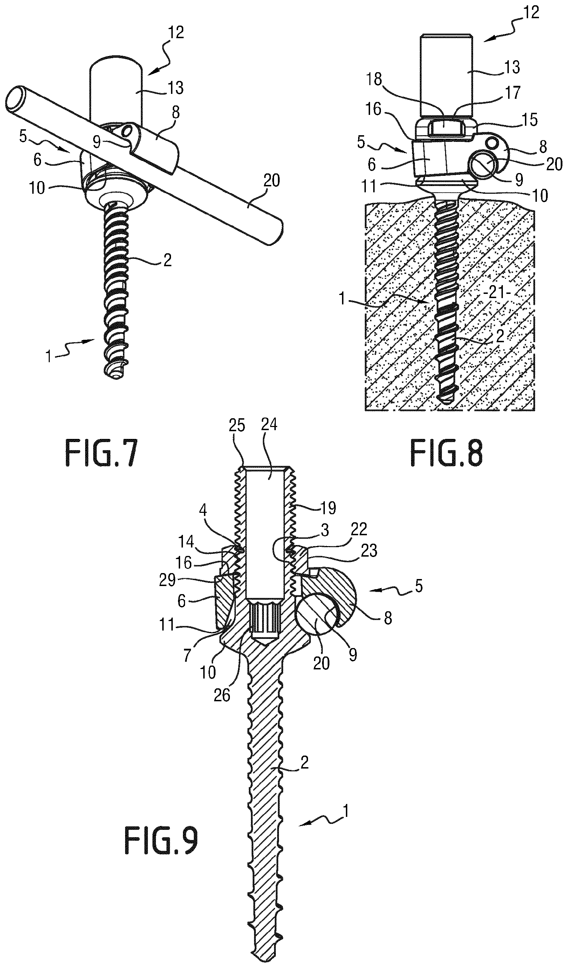

Un écrou 12 vient compléter l'ensemble vis 1-connecteur 5 pour le rendre opérationnel. Il se présente, dans l'exemple représenté, sous la forme d'un tube cylindrique 13 comportant sur au moins la partie inférieure de sa paroi interne un filetage 14 correspondant au filetage que porte le pion 3 de la vis 1, et, à sa partie inférieure, une collerette 15 qui est destinée à venir reposer sur la face supérieure 16 du connecteur 5 de façon à coincer, après vissage de l'écrou 12, le connecteur 5 contre le siège 10. On voit sur les figures 8 et 9 que dans leurs positions respectives définitives, les axes longitudinaux de la vis 1 et de la perforation 7 du connecteur 5 ne sont pas strictement alignés, du fait de la déviation du connecteur 5 imposée par la présence de la tige 20 du dispositif d'ostéosynthèse et son serrage contre le siège 10 et de la géométrie sphérique du contact connecteur 5-écrou 12, 22. Typiquement, l'angulation entre ces deux axes peut aller jusqu'à ± 15° dans toutes les directions de l'espace. Cette géométrie sphérique du contact connecteur 5-écrou 12, 22 permet également de limiter la déformation du crochet 8 du connecteur lors du serrage de la tige sur le siège 10.

Dans l'exemple non limitatif représenté sur les figures 4 à 8, l'écrou 12 comporte, à la jonction entre la collerette 15 et le tube cylindrique 13, une ligne de moindre résistance 17 sous forme d'une diminution localisée de l'épaisseur de la paroi externe du tube cylindrique 13. Elle permet, à la fin de la mise en place du dispositif d'ostéosynthèse, de séparer le tube 13 de la collerette 15 par un effort de torsion de façon à diminuer l'encombrement vertical de l'ensemble, comme on l'expliquera plus loin. Un pan 18 ménagé sur la collerette 15 procure un appui pour un outil avec lequel cette séparation peut être effectuée. La séparation de la partie supérieure sécable 19 du pion 3 est généralement effectuée dans le même mouvement, au moyen d'un effort de flexion.

De façon classique, l'ensemble dont on vient de décrire les pièces séparées est conçu pour, conformément à ce que l'on voit sur les figures 7 et 8, lorsque l'écrou 12 est serré, le crochet 8 du connecteur qui reçoit la tige 20 du dispositif d'ostéosynthèse presse cette tige 20 contre le siège 10 de la vis 1, celle-ci étant insérée dans un os 21 représenté sur la figure 8. La figure 9 montre de façon plus explicite, en coupe longitudinale, ce même ensemble, muni ici d'un écrou 22 de forme plus habituelle, qui se distingue de l'écrou 12 représenté sur les autres figures en ce qu'il est dépourvu de portion tubulaire 13 et présente une surface externe 23 pourvue de pans permettant son serrage par un tournevis qui vient le coiffer. Cet écrou 22 présente un filetage interne 14 correspondant à celui du pion 3, tout comme l'écrou 12 précédent. La hauteur de l'écrou 22 est conçue pour que, lorsqu'il est dans la position représentée sur la figure 9 et assure le blocage de l'ensemble vis 1-connecteur 5-tige 20, la ligne de moindre résistance 4 du pion 3 émerge légèrement au-dessus de l'écrou 22, de sorte que le pion 3 puisse être raccourci par brisure le long de la ligne de moindre résistance 4.

Sur la figure 9, on voit ce qui constitue une caractéristique essentielle de l'invention. Cette caractéristique est que le pion 3 est creux, en ce qu'il présente sur toute sa hauteur un espace intérieur 24 qui débouche sur l'extrémité supérieure 25 du pion 3. Cet espace intérieur 24 comporte dans sa partie inférieure une empreinte 26 munie de pans ou, comme représenté, en forme d'étoile (du type communément appelé « Torx^{®} »), dans laquelle peut venir s'insérer l'extrémité d'un tournevis de forme et dimensions correspondantes, manipulable par le chirurgien. Cette insertion peut avoir lieu à tout moment puisque l'espace intérieur 24 du pion 3 est en permanence accessible depuis l'extérieur. Bien entendu, d'autres configurations d'empreintes, de fonction équivalente, peuvent être employées.

Dans l'exemple représenté sur les figures, cette empreinte 26, en coopération avec le tournevis, est la seule possibilité qui existe, pour le chirurgien, de faire tourner la vis 1, notamment lors de la mise en place de la vis 1 sur l'os 21. En variante, comme on l'a dit plus haut, cette fonction de rotation de la vis 3 lors de sa mise en place peut aussi être assurée de façon classique par un ensemble de pans ménagés sur la surface externe du pion 3, sur lesquels vient en prise un tournevis. L'empreinte intérieure 26 du pion 3 est utilisée lorsque le chirurgien désire modifier l'enfoncement de la vis 3 dans l'os 21, par exemple pour ajuster la distance entre la surface de l'os 21 et la tige 20.

C'est dans ce genre de circonstances que l'invention est utile. En effet, la permanence de l'accès à l'empreinte 26 permet au chirurgien de modifier cet enfoncement à tout moment, même après la complète mise en place du dispositif d'ostéosynthèse rachidienne, incluant la mise en place du connecteur 5 et de l'écrou 12 ou 22 qui masquent alors les éventuels pans du pion 3 et les rendent inutilisables, et le serrage des écrous 12 ou 22 et, lorsqu'elles sont présentes sur les pièces d'origine, la rupture selon leurs lignes de moindre résistance 4, 17 des parties supérieures du pion 3 et de l'écrou 12.

A cet effet, le chirurgien doit souvent commencer par desserrer plus ou moins l'écrou 12 ou 22 afin de diminuer la pression exercée par la tige 20 sur la collerette 10 de la vis 1 et la tension de l'ensemble du dispositif d'ostéosynthèse, mais sans forcément dégager la tige 20 de l'emprise du crochet 8. Ainsi, la vis 1 retrouve un certain degré de mobilité, qui permet par rotation la correction de sa position en hauteur. Après quoi, l'écrou 12 ou 22 peut être resserré, et le dispositif d'ostéosynthèse se retrouve donc à nouveau bloqué et remis en tension, dans sa position corrigée, après, éventuellement, qu'une opération similaire a été effectuée sur une ou plusieurs autres vis 1 du dispositif.

L'espace intérieur 24 du pion 3 est avantageusement utilisable également lors de l'opération de réduction, c'est-à-dire lorsque les positions relatives des vertèbres sont modifiées par le chirurgien. Celui-ci peut enfoncer dans ledit espace intérieur 24 du pion 3 un outil lui permettant de manipuler la vertèbre concernée et de la mettre dans la position souhaitée

Un autre avantage de l'empreinte 26 selon l'invention est qu'elle rend la vis 1 également manipulable lorsque la partie sécable 19 du pion 3 s'est rompue accidentellement pendant la mise en place du dispositif. Comme la manipulation de la vertèbre lors de la réduction s'effectue de préférence, selon l'invention, par l'intérieur du pion 3 et non par son extérieur, ce risque de rupture prématurée est réduit par rapport à l'art antérieur. Il ne peut cependant pas être totalement supprimé, mais la présence de l'empreinte 26 permet de diminuer sensiblement les inconvénients qui seraient liés à cette rupture en conservant une bonne possibilité de mise en prise à la partie subsistante du pion 3.

De plus, dans le cas d'un écrou 12 présentant une portion tubulaire 13, la partie sécable du pion 3 est protégée tant que ladite portion tubulaire 13 n'a pas été séparée du restant de l'écrou 12, ce qui constitue une limitation supplémentaire du risque de rupture prématurée du pion 3.

Ce que l'on vient de dire s'applique également au cas où les vis 1 ont d'autres utilisations que l'ostéosynthèse rachidienne, par exemple lorsqu'elles sont intégrées à un dispositif de réduction d'une fracture d'un os long.

Dans le cas où ou utilise un écrou 12 à partie supérieure tubulaire 13 éventuellement sécable, il est également avantageux de prévoir, comme représenté sur la figure 6, que l'espace intérieur 27 de l'écrou 12 comporte des pans 28 avec lesquels un tournevis peut être mis en prise lors du serrage de l'écrou 12.

Si on souhaite que ces pans 28 soient utilisables y compris après la section de la partie tubulaire 13 de l'écrou 12 pour assurer une préhension et une rotation facile de l'écrou 12 pour son desserrage puis son resserrage, ils doivent être présents jusqu'en-dessous de la ligne de moindre résistance 17 de façon à ce que l'écrou 12 soit serrable et déserrable par un tournevis à tous les stades de la mise en place et de l'utilisation du dispositif d'ostéosynthèse. Ce n'est pas le cas dans l'exemple représenté, voir la figure 6, où le déserrage de l'écrou 12 peut être assuré à l'aide du pan 18 ménagé sur la surface externe de la collerette 15 et d'un outil coopérant avec ce pan 18.

Les dispositifs qui ont été représentés en détail ne sont, bien entendu, que des exemples non limitatifs. En particulier, le connecteur 5 et le siège 10 de la vis 1 sur lequel il s'appuie peuvent avoir des formes différentes, de tout type connu, du moment qu'ils permettent un libre accès permanent à l'empreinte 26 située dans la partie inférieure de l'espace intérieur 24 du pion 3. On pourrait, par exemple, prévoir que le connecteur 5 soit constitué par une pince, destinée à entourer la tige 20 sur toute sa périphérie, sans contact direct entre la tige et la vis 1, le connecteur se chargeant de transmettre au siège 10 les efforts exercés sur la vis 1 par la tige 20. Egalement, on peut prévoir que le siège 10 et le connecteur 5 autorisent une orientation du connecteur 5 différente de celle représentée, où l'orientation générale du connecteur 5 serait perpendiculaire à l'axe longitudinal de la vis 1.

Les dimensions de l'empreinte 26 selon l'invention peuvent être conformes à un standard de forme et de dimensions prédéterminé, de manière à rendre possible l'utilisation d'un tournevis de type courant. Elles peuvent être aussi d'un type particulier, auquel cas un tournevis adapté sera utilement intégré à un kit comportant également au moins un ensemble vis 1 - connecteur 5 - écrou 12, voire également un ou plusieurs outils permettant de rompre le pion 3 et l'écrou 12 au niveau de leurs parties sécables respectives 4, 17 si elles existent. Le cas échéant, ce kit pourra également inclure un tournevis pour la manipulation de l'écrou (12, 22).

## Revendications

1. - Vis osseuse (1) comportant une partie inférieure d'ancrage osseux munie d'un filet (2), une partie supérieure constituée par un pion (3) à surface externe filetée pour la réception d'un écrou (12 ; 22), et un siège (10) pour la réception d'un connecteur (6) destiné à relier la vis (1) à une tige (20) d'un dispositif d'ostéosynthèse et à être maintenu contre le siège (10) par l'écrou (12 ; 22), **caractérisée en ce que** ledit pion (3) est creux et présente un espace intérieur (24) débouchant sur son extrémité supérieure (25), la partie inférieure dudit espace intérieur (24) se terminant par une empreinte (26) pour la réception d'un tournevis, et **en ce que** le pion (3) présente une ligne de moindre résistance (4) le rendant sécable.

2. - Ensemble formé par une vis osseuse (1), un connecteur (5) destiné à relier la vis (1) à une tige (20) d'un dispositif d'ostéosynthèse, et un écrou (12 ; 22) destiné à maintenir le connecteur (5) contre le siège (10) de la vis (1), **caractérisé en ce que** la vis osseuse (1) est du type selon la revendication 1.

3. - Ensemble selon la revendication 2, **caractérisé en ce que** l'écrou (12 ; 22) comporte dans sa partie inférieure une collerette (15) destinée à venir reposer sur la face supérieure (16) dudit connecteur (5) pour le coincer contre le siège (10) après vissage de l'écrou (12).

4. Ensemble selon l'une des revendications 2 ou 3, **caractérisé en ce que** le connecteur (5) comporte une surface supérieure (16) en forme de portion de sphère et **en ce que** la surface inférieure (29) de l'écrou (12 ; 22) est aussi en forme de portion de sphère.

5. Ensemble selon l'une des revendications 2 à 4, **caractérisé en ce que** l'écrou (12) comporte une partie supérieure tubulaire (13).

6. - Ensemble selon la revendication 5, **caractérisé en ce que** ladite partie tubulaire (13) de l'écrou (12) comporte une ligne de moindre résistance (17) la rendant sécable.

7. - Ensemble selon la revendication 5 ou 6, **caractérisé en ce que** l'espace intérieur (27) de l'écrou (12) comporte des pans (28) avec lesquels un tournevis peut être mis en prise lors du serrage et du desserrage de l'écrou (12).

8. - Ensemble selon les revendications 6 et 7 prises ensemble, **caractérisé en ce que** lesdits pans subsistent sur l'espace intérieur (27) de l'écrou (12) après la section de sa partie supérieure tubulaire (13) selon la ligne de moindre résistance (17).

9. - Kit pour dispositif d'ostéosynthèse, du type comportant au moins un ensemble vis (1) - connecteur (5) - écrou (12 ; 22), **caractérisé en ce qu'**au moins une vis est du type selon la revendication 1, et **en ce qu'**il comporte également un tournevis destiné à venir en prise avec ladite empreinte (26) terminant la partie inférieure de l'espace intérieur (24) du pion (3).

10. - Kit selon la revendication 9, **caractérisé en ce que** ledit écrou (12) est du type selon la revendication 7, et **en ce que** ledit kit comporte également un tournevis destiné à venir en prise avec les pans (28) de l'espace intérieur (27) de l'écrou (12).

## Patentansprüche

1. Knochenschraube (1), umfassend einen unteren Abschnitt zur Knochenverankerung, der mit einem Gewinde (2) versehen ist, einen oberen Abschnitt, der aus einem Stift (3) mit einer Außenfläche mit Gewinde zum Aufnehmen einer Mutter (12; 22) besteht, und einen Sitz (10) zum Aufnehmen eines Verbinders (6), der dazu bestimmt ist, die Schraube (1) mit einem Schaft (20) einer Osteosynthesevorrichtung zu verbinden und durch die Mutter (12; 22) gegen den Sitz (10) gehalten zu werden, **dadurch gekennzeichnet, dass** der Stift (3) hohl ist und einen Innenraum (24) aufweist, der an seinem oberen Ende (25) mündet, wobei der untere Abschnitt des Innenraums (24) in einer Vertiefung (26) zum Aufnehmen eines Schraubendrehers endet, und dass der Stift (3) eine Linie des geringsten Widerstands (4) aufweist, die ihn trennbar macht.

2. Anordnung, gebildet durch eine Knochenschraube (1), einen Verbinder (5), der dazu dient, die Schraube (1) mit einem Schaft (20) einer Osteosynthesevorrichtung zu verbinden, und eine Mutter (12; 22) zum Halten des Verbinders (5) gegen den Sitz (10) der Schraube (1), **dadurch gekennzeichnet, dass** die Knochenschraube (1) von der Art nach Anspruch 1 ist.

3. Anordnung nach Anspruch 2, **dadurch gekennzeichnet, dass** die Mutter (12; 22) in ihrem unteren Abschnitt einen Bund (15) aufweist, der dazu bestimmt ist, an der Oberseite (16) des Verbinders (5) anzuliegen, um ihn nach dem Aufschrauben der Mutter (12) gegen den Sitz (10) zu klemmen.

4. Anordnung nach einem der Ansprüche 2 oder 3, **dadurch gekennzeichnet, dass** der Verbinder (5) eine obere Fläche (16) in Form eines Kugelabschnitts aufweist und dass die untere Fläche (29) der Mutter (12; 22) auch in Form eines Kugelabschnitts ist.

5. Anordnung nach einem der Ansprüche 2 bis 4, **dadurch gekennzeichnet, dass** die Mutter (12) einen rohrförmigen oberen Abschnitt (13) aufweist.

6. Anordnung nach Anspruch 5, **dadurch gekennzeichnet, dass** der rohrförmige Abschnitt (13) der Mutter (12) eine Linie (17) des geringsten Widerstands aufweist, die ihn trennbar macht.

7. Anordnung nach Anspruch 5 oder 6, **dadurch gekennzeichnet, dass** der Innenraum (27) der Mutter (12) Ansätze (28) aufweist, mit denen ein Schraubendreher beim Anziehen und Lösen der Mutter (12) in Eingriff gebracht werden kann.

8. Anordnung nach den Ansprüchen 6 und 7 zusammengenommen, **dadurch gekennzeichnet, dass** die Ansätze auf dem Innenraum (27) der Mutter (12) verbleiben, nachdem ihr rohrförmiger oberer Abschnitt (13) entlang der Linie des geringsten Widerstands (17) durchtrennt wurde.

9. Kit für Osteosynthesevorrichtung, von der Art, die mindestens eine Anordnung aus Schraube (1) - Verbinder (5) - Mutter (12; 22) umfasst, **dadurch gekennzeichnet, dass** mindestens eine Schraube von der Art nach Anspruch 1 ist, und dadurch, dass es auch einen Schraubendreher umfasst, der dazu bestimmt ist, mit der Vertiefung (26) in Eingriff zu kommen, die den unteren Abschnitt des Innenraums (24) des Stifts (3) abschließt.

10. Kit nach Anspruch 9, **dadurch gekennzeichnet, dass** die Mutter (12) von der Art nach Anspruch 7 ist, und dass das Kit auch einen Schraubendreher umfasst, der dazu bestimmt ist, mit den Flanken (28) des Innenraums (27) der Mutter (12) in Eingriff zu kommen.

## Claims

1. Bone screw (1) comprising a lower bone anchoring part provided with a thread (2), an upper part constituted by a pin (3) with a threaded external surface for receiving a nut (12; 22), and a seat (10) for receiving a connector (6) intended to connect the screw (1) to a rod (20) of an osteosynthesis device and to be held against the seat (10) by the nut (12; 22), **characterized in that** said pin (3) is hollow and has an internal space (24) opening onto its upper end (25), the lower part of said internal space (24) terminating in an indentation (26) for receiving a screwdriver, and **in that** the pin (3) has a line of least resistance (4) making it breakable.

2. An assembly formed by a bone screw (1), a connector (5) for connecting the screw (1) to a rod (20) of an osteosynthesis device, and a nut (12; 22) for holding the connector (5) against the seat (10) of the screw (1), **characterized in that** the bone screw (1) is of the type according to claim 1.

3. Assembly according to claim 2, **characterised in that** the nut (12; 22) comprises in its lower part a collar (15) intended to rest on the upper face (16) of the said connector (5) in order to wedge it against the seat (10) after the nut (12) has been screwed on.

4. An assembly according to one of claims 2 or 3, **characterised in that** the connector (5) has an upper surface (16) in the form of a portion of a sphere and **in that** the lower surface (29) of the nut (12; 22) is also in the form of a portion of a sphere.

5. An assembly according to one of claims 2 to 4, **characterised in that** the nut (12) comprises a tubular upper part (13).

6. An assembly according to claim 5, **characterised in that** said tubular portion (13) of the nut (12) has a line of least resistance (17) making it breakable.

7. An assembly according to claim 5 or 6, **characterised in that** the inner space (27) of the nut (12) has flats (28) with which a screwdriver can be engaged when tightening and loosening the nut (12).

8. An assembly according to claims 6 and 7 taken together, **characterised in that** said flats remain on the inner space (27) of the nut (12) after the section of its tubular upper part (13) along the line of least resistance (17).

9. Kit for an osteosynthesis device, of the type comprising at least one screw (1) - connector (5) - nut (12; 22) assembly, **characterised in that** at least one screw is of the type according to claim 1, and **in that** it also comprises a screwdriver intended to engage with the said impression (26) terminating the lower part of the inner space (24) of the pin (3).

10. A kit according to claim 9, **characterised in that** said nut (12) is of the type according to claim 7, and **in that** said kit also comprises a screwdriver for engaging the flats (28) of the inner space (27) of the nut (12).
